# EUROPEAN PATENT APPLICATION

(11) **EP 4 702 916 A1**
(43) Date of publication of application: **04.03.2026**
(21) Application number: 25197159.4
(22) Date of filing: 20.08.2025
(51) Int. Cl.: A61B 5/00, A61B 5/08, A61B 5/11, A61B 5/113

(54) **MEDICAL IMAGE CAPTURING SUPPORT APPARATUS, OPERATION METHOD OF MEDICAL IMAGE CAPTURING SUPPORT APPARATUS, PROGRAM, AND MEDICAL IMAGE CAPTURING APPARATUS**

(30) Priority: 27.08.2024 JP 2024145998
(71) Applicant: FUJIFILM Corporation, Tokyo 106-8620 (JP)
(72) Inventor: HAMADA, Kota, Tokyo, 106-8620 (JP); KITAMOTO, Shizue, Tokyo, 1068620 (JP); UCHIO, Yoshiki, Tokyo, 1068620 (JP)
(74) Representative: Dehns Germany Partnerschaft mbB

(57) **Abstract**

Provided are a medical image capturing support apparatus, an operation method of a medical image capturing support apparatus, a program, and a medical image capturing apparatus that realize identification of a body position of a subject (103) with high accuracy. A medical image capturing support apparatus acquires a plurality of time-series captured images generated by capturing a subject (103), detects, from the plurality of time-series captured images, a body movement region of the subject (103) in the captured image in which a movement of the subject (103) having periodicity and magnitude corresponding to a respiratory movement of the subject (103) occurs, and identifies a body position of the subject (103) based on the body movement region.

## Description

### BACKGROUND OF THE INVENTION

### 1. Field of the Invention

The present disclosure relates to a medical image capturing support apparatus, an operation method of a medical image capturing support apparatus, a program, and a medical image capturing apparatus.

### 2. Description of the Related Art

In a typical MRI examination, a user sets imaging conditions such as a body position of a patient in advance, and then places the patient on an examination bed in a shielded room and starts a scan, thereby starting the examination. The user represents a person who operates the MRI apparatus, such as a technician, and a user or an operator. The patient is a person to be examined, and can be referred to as an examinee, a subject, an examination subject, and the like. MRI is an abbreviation for Magnetic Resonance Imaging.

JP2014-121364A discloses an X-ray CT apparatus that notifies that a body position of a subject detected using a camera is different from a body position of the subject set in advance. The apparatus disclosed in JP2014-121364A identifies an orientation, a body position, and the like of the subject in a craniocaudal direction. In a case where the identified body position and the like of the subject are inappropriate, the apparatus displays inappropriate conditions and a message prompting a change in setting to appropriate conditions. It should be noted that CT is an abbreviation for Computed Tomography. In addition, identification in the present specification is synonymous with recognition disclosed in JP2014-121364A.

### SUMMARY OF THE INVENTION

However, in a case where image processing is performed on a captured image of a subject obtained by imaging the subject using a camera and a body position of the subject is identified based on a result of the image processing, sufficient accuracy in identification may not be obtained, and it is desired to realize identification of the body position of the subject with higher accuracy.

The present disclosure has been made in view of such circumstances, and an object of the present disclosure is to provide a medical image capturing support apparatus, an operation method of a medical image capturing support apparatus, a program, and a medical image capturing apparatus that realize identification of a body position of a subject with high accuracy.

According to a first aspect of the present disclosure, there is provided a medical image capturing support apparatus comprising: a processor; and a memory in which a program to be executed by the processor is stored, in which the processor is configured to acquire a plurality of time-series captured images generated by capturing a subject, detect, from the plurality of time-series captured images, a body movement region of the subject in the captured image in which a movement of the subject having periodicity and magnitude corresponding to a respiratory movement of the subject occurs, and identify a body position of the subject based on the body movement region.

With the medical image capturing support apparatus according to the first aspect, the body movement region in the captured image of the subject is detected, and the body position of the subject is identified based on the body movement region. As a result, the body position of the subject is identified with high accuracy.

An example of the body position of the subject includes an orientation of the subject relative to a moving direction of an examination bed. Examples of the orientation of the subject include head-first and foot-first.

The plurality of time-series captured images may be frames of a moving image, or may be a plurality of static images successive in time series.

According to a first embodiment, in the medical image capturing support apparatus according to the first aspect, the processor may be configured to detect at least one of a chest or an abdomen of the subject in the captured image based on the body movement region.

According to a second embodiment, in the medical image capturing support apparatus according to the first aspect or first embodiment, the processor may be configured to detect the body movement region by applying optical flow calculation processing of calculating an optical flow in the plurality of time-series captured images.

According to a third embodiment, in the medical image capturing support apparatus according to any one of the first aspect or first to second embodiment, the processor may be configured to acquire a plurality of time-series first captured images obtained by capturing the subject using a first camera disposed on one side of an imaging space, detect a first body movement region as the body movement region from the plurality of time-series first captured images, acquire a plurality of time-series second captured images obtained by capturing the subject using a second camera disposed on the other side of the imaging space, detect a second body movement region as the body movement region from the plurality of time-series second captured images, and identify the body position of the subject according to a comparison result between the first body movement region and the second body movement region.

According to a fourth embodiment, in the medical image capturing support apparatus according to any one of the first aspect or first to third embodiment, the processor may be configured to acquire examination site information representing an examination site, specify a position of the body movement region in the captured image with reference to the examination site information, and identify the body position of the subject according to the position of the body movement region in the captured image.

According to a fifth embodiment, in the medical image capturing support apparatus according to any one of the first aspect or first to fourth embodiment, the processor may be configured to acquire a respiratory signal representing a respiratory movement of the subject from a respiratory sensor, and detect the body movement region based on a comparison result between periodicity of the respiratory signal and periodicity of a region in the captured image where a movement of the subject having periodicity occurs.

According to a sixth embodiment, in the medical image capturing support apparatus according to any one of the first aspect or first to fifth embodiment, the processor may be configured to identify whether the subject moving to an imaging position is head-first or foot-first, as the body position of the subject.

According to a second aspect of the present disclosure, there is provided an operation method of a medical image capturing support apparatus, the operation method comprising causing a computer functioning as the medical image capturing support apparatus to execute: acquiring a plurality of time-series captured images generated by capturing a subject; detecting, from the plurality of time-series captured images, a body movement region of the subject in the captured image in which a movement of the subject having periodicity and magnitude corresponding to a respiratory movement of the subject occurs; and identifying a body position of the subject based on the body movement region.

According to the operation method of a medical image capturing support apparatus according to the second aspect of the present disclosure, it is possible to obtain the same actions and effects as those of the medical image capturing support apparatus according to the first aspect. Configuration requirements of the medical image capturing support apparatus according to the first to sixth embodiments can be applied as configuration requirements of the operation method of a medical image capturing support apparatus according to other aspects.

According to a third aspect of the present disclosure, there is provided a program for causing a computer functioning as a medical image capturing support apparatus to realize: a function of acquiring a plurality of time-series captured images generated by capturing a subject; a function of detecting, from the plurality of time-series captured images, a body movement region of the subject in the captured image in which a movement of the subject having periodicity and magnitude corresponding to a respiratory movement of the subject occurs; and a function of identifying a body position of the subject based on the body movement region.

With the program according to the third aspect of the present disclosure, it is possible to obtain the same actions and effects as those of the medical image capturing support apparatus according to the first aspect. Configuration requirements of the medical image capturing support apparatus according to the first to sixth embodiments can be applied as configuration requirements of a program according to other aspects.

According to a fourth aspect of the present disclosure, there is provided a medical image capturing apparatus comprising: an imaging unit configured to image a subject and generate imaging data of the subject; an image reconstruction unit configured to generate reconstruction based on the imaging data; a processor; and a memory in which a program to be executed by the processor is stored, in which the processor is configured to acquire a plurality of time-series captured images generated by capturing the subject, detect, from the plurality of time-series captured images, a body movement region of the subject in the captured image in which a movement of the subject having periodicity and magnitude corresponding to a respiratory movement of the subject occurs, and identify a body position of the subject based on the body movement region.

According to the medical image capturing apparatus according to the fourth aspect of the present disclosure, it is possible to obtain the same actions and effects as those of the medical image capturing support apparatus according to the first aspect. Configuration requirements of the medical image capturing support apparatus according to the first to sixth embodiments can be applied as configuration requirements of the operation method of a medical image capturing apparatus according to other aspects.

According to the present disclosure, the body movement region in the captured image of the subject is detected, and the body position of the subject is identified based on the body movement region. As a result, the body position of the subject is identified with high accuracy.

### BRIEF DESCRIPTION OF THE DRAWINGS

Fig. 1 is a schematic configuration diagram of a medical image capturing system according to a first embodiment.
Fig. 2 is a block diagram showing a specific example of a signal analysis unit shown in Fig. 1.
Fig. 3 is a block diagram showing an example of a hardware configuration of a signal processing device shown in Figs. 1 and 2.
Fig. 4 is a flowchart of a body position identification method according to the first embodiment.
Fig. 5 is an explanatory diagram of a captured image obtained by capturing a subject using a first camera.
Fig. 6 is a schematic diagram showing arrangement of a first camera and a second camera provided in an MRI apparatus according to a second embodiment.
Fig. 7 is an explanatory diagram of a captured image obtained by capturing the subject using the second camera.
Fig. 8 is a schematic configuration diagram of a medical image capturing system according to a third embodiment.
Fig. 9 is a flowchart of a body position identification method according to the third embodiment.
Fig. 10 is an explanatory diagram of a captured image obtained by capturing the subject undergoing a head examination using the first camera.
Fig. 11 is an explanatory diagram of a captured image obtained by capturing the subject undergoing a pelvic region examination using the first camera.
Fig. 12 is an explanatory diagram of a captured image obtained by capturing the subject undergoing the head examination using the second camera.
Fig. 13 is an explanatory diagram of a captured image obtained by capturing the subject undergoing the pelvic region examination using the second camera.
Figs. 14A and 14B are explanatory diagrams of a captured image obtained by capturing the subject undergoing an abdominal examination using the first camera and the second camera.
Fig. 15 is a schematic configuration diagram of a medical image capturing system according to a fourth embodiment.
Fig. 16 is a flowchart of a body position identification method according to the fourth embodiment.
Fig. 17 is an explanatory diagram showing a waveform of a respiratory signal acquired using a respiratory sensor.
Figs. 18A and 18B are explanatory diagrams of a captured image of the subject.
Fig. 19 is a schematic diagram showing a period of an optical flow.

### DESCRIPTION OF THE PREFERRED EMBODIMENTS

Hereinafter, a preferred embodiment of the present invention will be described in detail with reference to the accompanying drawings. In the following description and the accompanying drawings, the same constituent elements are denoted by the same reference numerals, and the duplicated description thereof is omitted. In addition, in the following embodiment, in a case where a plurality of constituent elements are described and listed, it can be interpreted that at least one of the plurality of constituent elements is included.

### First Embodiment

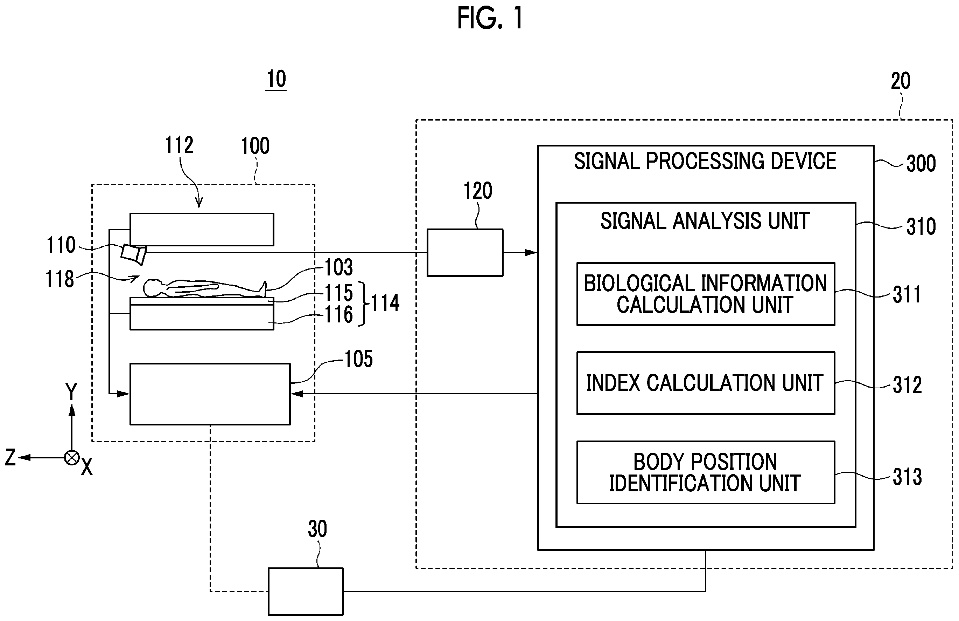

Fig. 1 is a schematic configuration diagram of a medical image capturing system according to a first embodiment. A medical image capturing system 10 comprises an MRI apparatus 100 and a biological information measurement system 20. The biological information measurement system 20 comprises a biological signal measurement device 120 and a signal processing device 300.

The MRI apparatus 100 comprises a gantry 112, which is an imaging apparatus main body, and an examination bed 114. The gantry 112 includes a cylindrical imaging space 118 called a bore. In the gantry 112, a static magnetic field generating magnet, a gradient magnetic field coil, and various other coils that generate a magnetic field in the imaging space 118 are disposed.

The examination bed 114 comprises a top plate 115 and a leg part 116 that supports the top plate 115. The top plate 115 can enter the imaging space 118 and can exit from the imaging space 118 by using a drive mechanism (not shown) provided in the leg part 116. The examination bed 114 may be configured to be fixed to the gantry 112, or may be a mobile dockable examination bed that is attachable to and detachable from the gantry 112.

In the MRI apparatus 100, three apparatus axes, which are three-dimensional orthogonal coordinate axes of a real space including the imaging space 118, are defined. A direction of each axis of the three apparatus axes is uniquely determined from the gantry 112 and the examination bed 114. A Z direction in Fig. 1 is a static magnetic field direction, a Y direction is a vertical direction, and an X direction is a direction orthogonal to the Y direction and the Z direction.

The MRI apparatus 100 can perform imaging by using biological information measured by using the biological signal measurement device 120. Specifically, the MRI apparatus 100 performs synchronous imaging using biological information such as pulsation and a respiratory movement. In addition, the MRI apparatus 100 performs correction of measurement data using biological information.

The MRI apparatus 100 comprises a control device 105. A computer comprising a processor and a memory in which a program to be executed by the processor is stored is applied as the control device 105.

The control device 105 performs various types of control such as imaging control for a medical image in the MRI apparatus 100 and movement control for the examination bed 114. The control device 105 transmits signals representing various types of information in the MRI apparatus 100 to a display device 30, and causes the display device 30 to display various types of information.

The control device 105 may acquire various types of information transmitted from the signal processing device 300. The control device 105 may perform imaging control for the medical image using various types of information transmitted from the signal processing device 300.

The MRI apparatus 100 is an example of a medical image capturing apparatus comprising an imaging unit that generates imaging data of a subject of the present disclosure and an image reconstruction unit that generates reconstruction based on the imaging data. The medical image may be a two-dimensional reconstructed image or a three-dimensional reconstructed image.

The biological signal measurement device 120 acquires biological information in a noncontact manner from a subject 103 under examination in which the MRI apparatus 100 is used. The biological signal measurement device 120 acquires a captured image of the subject 103 acquired from a first camera 110 as the biological information, and transmits the captured image to the signal processing device 300. The signal processing device 300 identifies a body position of the subject 103 by using the captured image of the subject 103.

The first camera 110 need only be disposed in a shielded room. For example, the first camera 110 may be disposed on a bore side inside the gantry 112 shown in Fig. 1. The first camera 110 may be disposed at a position on a rear side of the gantry 112. The first camera 110 may be a device capable of acquiring a captured image of the subject 103 other than the above. It is preferable that the first camera 110 comprises a fisheye lens and is configured to image the whole body of the subject 103.

The biological signal measurement device 120 may acquire, from a sensor such as a camera, an infrared ray, or a distance sensor, a signal representing a distance between the sensor and the subject 103 as a biological information signal, and may transmit the acquired biological information signal to the signal processing device 300. The distance sensor may be an electromagnetic wave sensor such as a millimeter wave sensor or may be an ultrasonic wave.

The signal processing device 300 may generate various types of information related to the subject 103 to be used for capturing the medical image of the subject 103 based on the biological information signal acquired from the biological signal measurement device 120.

The signal processing device 300 applies an image processing technology to the captured image of the subject 103 acquired by using the biological signal measurement device 120, and detects a body movement region in the subject 103 that significantly moves in a periodic manner. The signal processing device 300 identifies the body position of the subject 103 by using the fact that the body movement region in the subject 103 that significantly moves in a periodic manner is generally an upper body portion of the subject 103 where a respiratory movement occurs. For example, whether the body position of the subject 103 is head-first or foot-first is identified.

Here, the head-first refers to a body position in which the subject is transported into the imaging space head first. The foot-first refers to a body position in which the subject is transported into the imaging space foot first.

An example of the image processing on the captured image of the subject 103 is optical flow calculation processing of detecting a movement of an object between two time-series captured images and calculating an optical flow representing the movement of the object as a velocity vector.

The signal processing device 300 acquires a movement vector amount representing the magnitude of the movement of the subject 103 for a region where the movement of the subject 103 occurs significantly. The signal processing device 300 may acquire an area of the region where the movement of the subject 103 occurs significantly and a combination thereof.

The signal processing device 300 comprises a signal analysis unit 310. The signal analysis unit 310 comprises a biological information calculation unit 311, an index calculation unit 312, and a body position identification unit 313. The biological information calculation unit 311 acquires moving images to which a specified frame rate is applied, which are captured images of the subject 103 transmitted from the biological signal measurement device 120, and detects the movement of the subject 103 based on the movement of the object between temporally adjacent frame images.

The index calculation unit 312 calculates an index value representing the magnitude of the movement of the subject 103. The index calculation unit 312 may calculate an index value of a region where the subject 103 moves significantly and a combination thereof. An example of the index value of the region where the subject 103 moves significantly is the area of the region.

The body position identification unit 313 detects the body movement region in the subject 103 that significantly moves in a periodic manner, based on the index value for the subject 103 calculated by using the index calculation unit 312. For example, as the body movement region in the subject 103 that significantly moves in a periodic manner, at least one of a chest or an abdomen where a respiratory movement occurs due to breathing may be detected. In a case where a plurality of region in which periodic movements occur are detected, a region in which the periodic movement is greatest may be set as the body movement region.

The body position identification unit 313 identifies whether the body position of the subject 103 is head-first or foot-first based on the body movement region in the captured image of the subject 103 in which a movement having periodicity and magnitude corresponding to the respiratory movement occurs.

The signal processing device 300 transmits a signal representing the body position of the subject 103 to the display device 30. The display device 30 displays information representing the body position of the subject 103. The display device 30 may display character information representing the body position of the subject 103.

The display device 30 may also serve as a display device provided in the biological information measurement system 20, or may also serve as a display device provided in the MRI apparatus 100. The display device 30 may be a device independent of the biological information measurement system 20 and the MRI apparatus 100.

The medical image capturing system 10 may comprise an input device. The input device may include a keyboard, a mouse, and the like. The input device may also serve as the display device 30 as a touch panel type display.

### Specific Example of Signal Analysis Unit

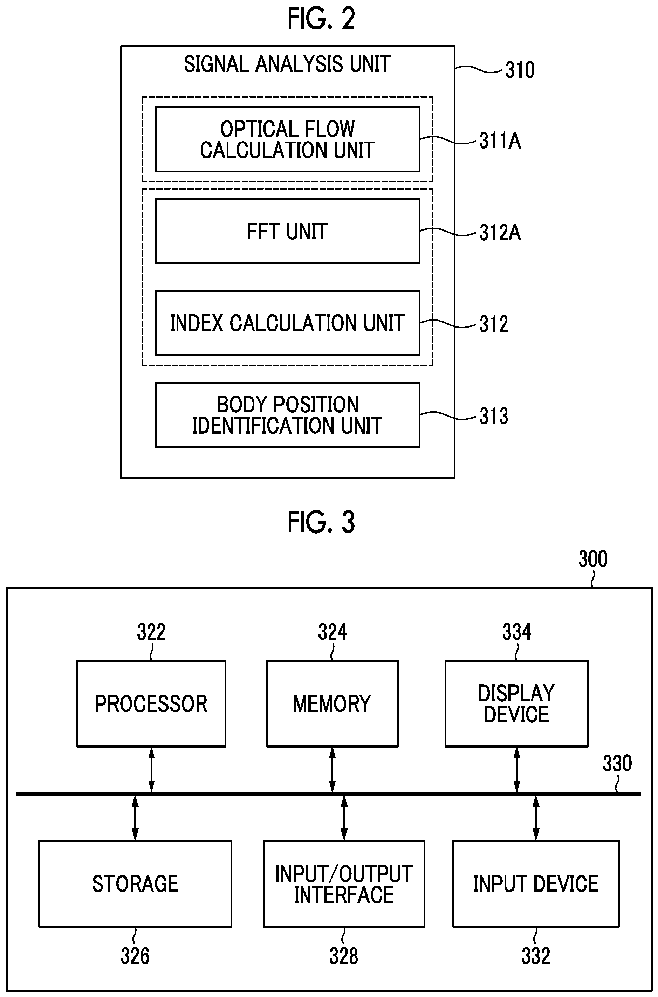

Fig. 2 is a block diagram showing a specific example of the signal analysis unit shown in Fig. 1. The signal analysis unit 310 comprises an optical flow calculation unit 311A as the biological information calculation unit 311 shown in Fig. 1.

The optical flow calculation unit 311A acquires a moving image as a captured image of the subject 103 captured by using the first camera 110. The captured image of the subject 103 may be a plurality of time-series static images. The imaging time of the subject 103 need only be equal to or longer than two typical respiratory cycles. For example, the imaging time of the subject 103 may be 10 seconds or more. Here, the term "image" in the present specification may include the meanings of an image signal and image data representing an image.

The optical flow calculation unit 311A calculates an optical flow representing a change in position of each pixel for two frame images adjacent in time. The optical flow calculation unit 311A may calculate an optical flow for each sub-pixel including a plurality of pixels adjacent to each other.

The optical flow calculation unit 311A sequentially switches between frame images in time series order for a plurality of time-series frame images, and calculates an optical flow for each combination of the frame images adjacent in time.

For example, an optical flow between a first frame image and a second frame image that are adjacent in time is calculated, and then an optical flow between the second frame image and a third frame image that are adjacent in time is calculated. The same processing is repeatedly performed on the frame images after the third frame image.

The index calculation unit 312 comprises an FFT unit 312A. The FFT unit 312A acquires periodicity information representing the periodicity of the optical flow in two frame images adjacent in time. It should be noted that FFT is an abbreviation for Fast Fourier Transform.

The index calculation unit 312 detects the body movement region of the subject 103 in the captured image. The body movement region is a region of the subject 103 where a movement occurs due to breathing of the subject 103. The captured image of the subject 103 here may be any of a plurality of frame images included in the moving image.

The body position identification unit 313 identifies whether the body position of the subject 103 is head-first or foot-first based on the body movement region of the subject 103 in the captured image calculated by the index calculation unit 312.

That is, the signal analysis unit 310 uses moving images, which are captured images of the subject 103 during an MRI examination, as input data, to detect a movement of an object between two successive frame images and calculates an optical flow representing the movement as a velocity vector.

Furthermore, the signal analysis unit 310 uses characteristics of a respiratory movement, which is a periodic movement of the subject 103, to specify the body movement region of the subject 103 in the captured image where the greatest movement occurs within a certain period of time, as a respiratory region. The signal analysis unit 310 can distinguish between the periodic body movement and the sudden body movement of the subject 103.

As described above, the signal analysis unit 310 specifies the body movement region of the subject 103 in the captured image, which significantly moves in a periodic manner due to the respiratory movement as the chest of the subject 103, and identifies whether the actual body position of the subject 103 is head-first or foot-first. The body movement region may be estimated to be the abdomen of the subject 103, and the body position of the subject 103 may be identified based on the estimation result.

### Hardware Configuration of Signal Processing Device

Fig. 3 is a block diagram showing an example of a hardware configuration of the signal processing device shown in Figs. 1 and 2. Various processes of the signal processing device 300 are realized by applying any computer. Any computer may have a processor that executes a program to execute various processes of the signal processing device 300.

Any computer may be a general-purpose computer such as a personal computer or a computer for a specific use such as a server computer. Any computer may be a system such as a workstation or may be other hardware elements capable of executing a program, such as a virtual machine.

At least some of functions of the signal processing device 300 may be realized using cloud computing. At least some of the functions of the signal processing device 300 may be provided as SaaS. It should be noted that SaaS is an abbreviation for Software as a Service.

The signal processing device 300 comprises a processor 322, a memory 324 as a main memory, a storage 326 as an auxiliary memory, an input/output interface 328, and a bus 330.

The processor 322 is connected to the memory 324, the storage 326, the input/output interface 328, an input device 332, and a display device 334 via the bus 330.

The memory 324 includes a RAM. The memory 324 may include a ROM. The storage 326 may be, for example, a hard disk drive, a solid state drive, or a combination of a plurality of these. In addition, the storage 326 may include an external storage device such as a removable medium.

It should be noted that RAM is an abbreviation for Random Access Memory, and ROM is an abbreviation for Read Only Memory. The hard disk drive may be referred to as HDD using an abbreviation for Hard Disk Drive. The solid state drive may be referred to as an SSD using an abbreviation for Solid State Drive.

A program, data, and the like for realizing various functions of the signal processing device 300 are stored in the storage device including the memory 324 and the storage 326. The processor 322 executes the programs stored in the memory 324 to realize various functions. The processor 322 integrally controls each unit of the signal processing device 300, various devices, units, and the like provided in the signal processing device 300, and performs various types of processing.

The input/output interface 328 includes a communication interface that is connectable to an electric communication line such as a local area network, a connection interface that is connectable to an external device, and the like. As the connection interface that is connectable to an external device, for example, a universal serial bus or HDMI (HDMI is a registered trademark) can be applied. It should be noted that HDMI is an abbreviation for High-Definition Multimedia Interface.

The processor 322 communicates with various devices of the signal processing device 300 via the input/output interface 328 to transmit and receive various types of information.

Examples of the input device 332 include a pointing device such as a keyboard and a mouse. The input device 332 may include a numeric keypad, various switch buttons, and the like. The input device 332 may include a voice input device. The input device 332 may be a touch panel type input device that is configured integrally with a display screen of the display device 334.

The display device 334 may be a liquid crystal display, an organic EL display, or a projector. The display device 334 may be an appropriate combination of a liquid crystal display and the like. Various types of information are displayed on the display device 334 in addition to the image captured by the signal processing device 300. The display device 334 is used as a part of the UI in a case of receiving an input via the input device 332. The present disclosure is not limited to one display device 334, and a form of a multi-display comprising a plurality of display devices can be used.

The display device 334 shown in Fig. 3 may be the display device 30 shown in Fig. 1. It should be noted that the organic EL may be referred to as OEL using an abbreviation for Organic Electro-Luminescence. UI is an abbreviation for User Interface.

In the present embodiment, each process is executed by any computer. In addition, any computer may be adapted to execute these processes using a processor, a program, or a combination of these. Any computer may be a general-purpose computer, a computer for a specific use, a system such as a workstation, or other hardware elements capable of executing a program.

One or a plurality of hardware may be applied to configure the processor 322, and the type of hardware is not limited. A programmable logic device such as a CPU, an MPU, and an FPGA may be applied as the hardware of the processor 322. A dedicated circuit that executes a specific process, such as an ASIC, may be applied as the processor 322. As the hardware of the processor 322, a GPU that performs a process specialized in image processing, an NPU that is specialized in AI processing, and the like may be applied.

The processor 322 functions as units, which are various processing units that execute various processes, and means, which are various processing means that execute various processes.

It should be noted that CPU is an abbreviation for Central Processing Unit, MPU is an abbreviation for Micro-Processing Unit, and FPGA is an abbreviation for Field-Programmable Gate Array. In addition, GPU is an abbreviation for Graphics Processing Unit, AI is an abbreviation for Artificial Intelligence, and NPU is an abbreviation for Neural network Processing Unit.

The processor 322 may be configured by combining different types of hardware. As the hardware of the processor 322, an electric circuit (circuitry) in which electric circuit elements such as semiconductor elements are combined is applied.

In a case where a plurality of pieces of hardware execute any one or more processes of the processor 322, the plurality of pieces of hardware may be located in devices physically separated from each other, or may be disposed in the same device. The order of the processes executed by the processor 322 is not limited to the order disclosed in the present specification and may be changed as appropriate. The hardware is configured by using an electric circuit or the like in which circuit elements such as semiconductor elements are combined.

Further, the present embodiment may be realized by applying hardware, software, firmware, microcode, or a combination thereof. The software, the firmware, and the microcode are configured by applying a program. For example, the program may be a program module group, and the functions of the software and the like may be realized by applying a processor that executes each function.

The program may be, for example, a program code stored in one or a plurality of non-transitory computer-readable media such as a storage medium and a storage, and a plurality of code segments. The program may be divided and stored in a plurality of non-transitory computer-readable media present in devices physically separated from each other.

The program code or the code segment may represent any combination of a procedure, a function, a subprogram, a routine, a subroutine, a module, a software package, a class, an instruction, a data structure, or a program statement. The program code or the code segment may be connected to another code segment or a hardware circuit by transmitting and receiving information, data, an argument, a parameter, or memory contents.

### Procedure of Body Position Identification Method according to First Embodiment

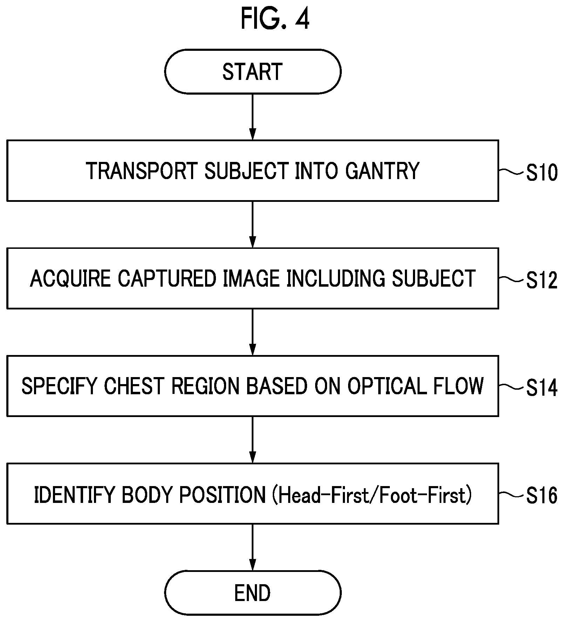

Fig. 4 is a flowchart of a body position identification method according to the first embodiment. The body position identification method shown in Fig. 4 is an example of an operation method of a medical image capturing support apparatus according to the present disclosure.

In step S10, the subject 103 placed on the examination bed 114 disposed on a front side of the gantry 112 is transported to the imaging space 118 in the gantry 112. That is, in step S10, an operator such as a technician operates a movement control device of the examination bed 114 to move the examination bed 114 on which the subject 103 lies on the top plate 115 from a specified position outside the gantry 112 to a predetermined position inside the imaging space 118 of the gantry 112 and to stop the examination bed 114. The front side of the gantry 112 can be referred to as the front of the gantry 112, the front surface side of the gantry 112, or the like.

In step S12, the imaging of the subject 103 placed on the examination bed 114 stopped at the specified position of the imaging space 118 corresponding to the examination site is started using the first camera 110, and the acquisition of the moving image of the subject 103 is started as the captured image. The captured image of the subject 103 may be referred to as a video signal of the subject 103.

The imaging of the subject 103 using the first camera 110 is performed before the imaging for acquiring the medical image of the subject 103 is started, and is ended before the imaging for acquiring the medical image of the subject 103 is started.

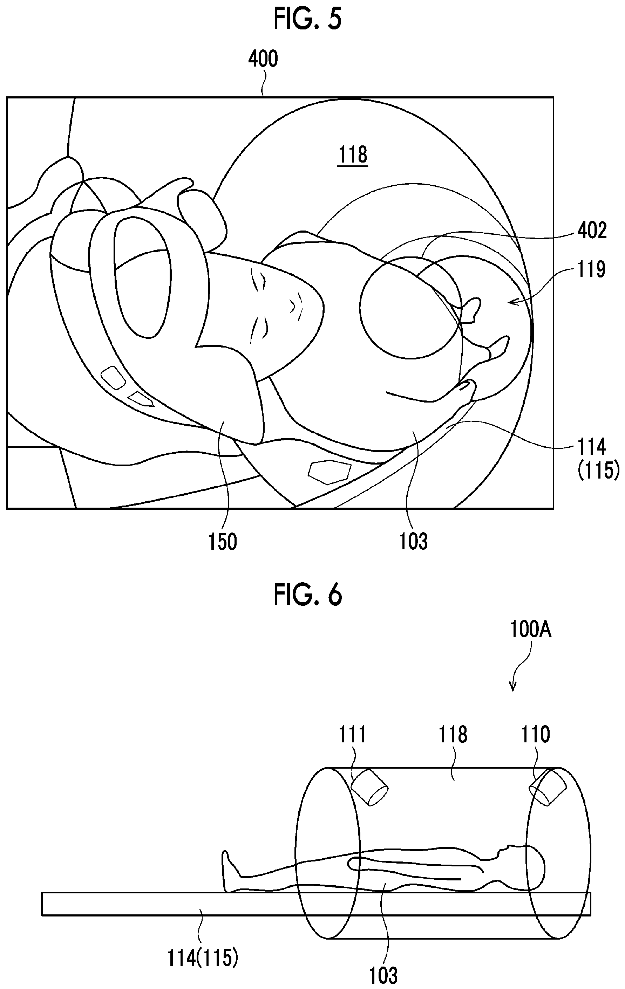

In step S14, the signal analysis unit 310 calculates the optical flow based on the captured image of the subject 103, and specifies at least one of a chest region or an abdomen region from the body movement region of the subject 103 in the captured image. Hereinafter, a case where the chest region is specified will be described. The chest region is denoted by reference numeral 402 and is shown in Fig. 5 and the like.

In step S16, the body position identification unit 313 identifies whether the body position of the subject 103 is head-first or foot-first based on the chest region in the captured image of the subject 103. The information on the body position of the subject 103 may be displayed on the display device 30. In a case where the body position of the subject 103 is identified, the procedure of the body position identification method is ended.

### Specific Example of Captured Image of Subject

Fig. 5 is an explanatory diagram of a captured image obtained by capturing the subject using the first camera. A left side of a first captured image 400 shown in Fig. 5 as viewed from the horizontal center is a rear side of the imaging space 118, and a right side of the first captured image 400 as viewed from the horizontal center is a front side of the imaging space 118. An entrance 119 of the imaging space 118 is shown at a right end of the first captured image 400.

The rear side of the imaging space 118 can be referred to as the rear side, the back side, or the far side of the imaging space 118. The front side of the imaging space 118 can be referred to as the front side, the front, or the front surface side of the imaging space 118. The position on the rear side of the imaging space 118 is an example of one side of an imaging space of the present disclosure.

The first captured image 400 shows the subject 103 with a receive coil for head 150 mounted on the head. The chest region 402 of the subject 103 in the first captured image 400 is specified using the signal analysis unit 310 shown in Fig. 1 in step S14 of Fig. 4.

The body position identification unit 313 identifies that the body position of the subject 103 is head-first based on the position of the chest region 402 of the subject 103 in the first captured image 400.

### Actions and Effects of First Embodiment

The signal processing device and the body position identification method according to the first embodiment can obtain the following actions and effects.
[1] Using the first camera 110 disposed on the rear side of the imaging space 118, a moving image is captured as the first captured image 400 of the subject 103 at a position where a medical image is captured. The optical flow is calculated from the first captured image 400 of the subject 103, and the chest region 402 of the subject 103 in the first captured image 400 is specified based on the optical flow.
   Whether the body position of the subject 103 is head-first or foot-first is identified based on the position of the chest region 402 of the subject 103 in the first captured image 400. As a result, the body position of the subject 103 is identified with high accuracy.
[2] From the first captured image 400 of the subject 103, the body movement region in which the movement having the periodicity and the magnitude corresponding to the respiratory movement of the subject 103 occurs is specified as the chest region 402. As a result, the characteristics of the respiratory movement that significantly moves in a periodic manner are used to specify the chest region 402 of the subject 103 with high accuracy.

In the present embodiment, capturing the moving image of the subject 103 is exemplified, but the image of the subject 103 may be captured as continuous static images to which a specified period is applied. The specified period need only be greater than twice the typical human respiratory cycle.

### Second Embodiment

Fig. 6 is a schematic diagram showing arrangement of a first camera and a second camera provided in an MRI apparatus according to a second embodiment. In an MRI apparatus 100A according to the second embodiment, a first camera 110 is disposed on the rear side of the imaging space 118, and a second camera 111 is disposed on the front side of the imaging space 118. The second camera 111 may be a camera comprising a fisheye lens, as with the first camera 110. The front side of the imaging space 118 is an example of the other side of the imaging space of the present disclosure.

The biological signal measurement device 120 shown in Fig. 1 acquires a first captured image, which is a moving image of the subject 103 captured by using the first camera 110, and a second captured image, which is a moving image of the subject 103 captured by using the second camera 111.

The signal processing device 300 acquires the first captured image and the second captured image from the biological signal measurement device 120. The signal processing device 300 calculates a first optical flow based on the first captured image and calculates a second optical flow based on the second captured image.

The body position identification unit 313 detects a first body movement region based on the first optical flow and detects a second body movement region based on the second optical flow. The body position identification unit 313 compares the first body movement region with the second body movement region and specifies the chest region 402 of the subject 103 based on the comparison result.

A procedure of a body position identification method according to the second embodiment is the same as the procedure of the body position identification method according to the first embodiment shown in Fig. 4, except for the processes in step S12, step S13, and step S14, as described above.

### Specific Example of Second Captured Image

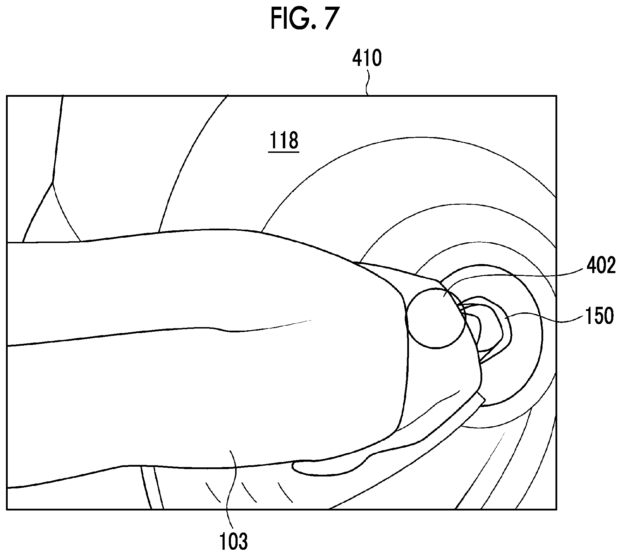

Fig. 7 is an explanatory diagram of a second captured image obtained by capturing the subject using the second camera. A second captured image 410 shown in Fig. 7 is generated by capturing the subject 103 shown in Fig. 6 using the second camera 111.

A left side from the horizontal center of the second captured image 410 is a front side of the imaging space 118, and a right side from the horizontal center of the second captured image 410 is a rear side of the imaging space 118.

The signal analysis unit 310 shown in Fig. 1 specifies a chest region 402 in the second captured image 410 from the second optical flow calculated based on the second captured image 410. The body position identification unit 313 compares the chest region 402 in the first captured image 400 shown in Fig. 5 with the chest region 402 in the second captured image 410 and identifies the body position of the subject 103 based on a more probable chest region.

The body position identification unit 313 may compare an area of the chest region 402 in the first captured image 400 with an area of the chest region 402 in the second captured image 410 and identify the body position of the subject 103 based on the captured image having a chest region with a relatively larger area.

In the examples shown in Figs. 5 and 7, the chest region 402 shown in Fig. 5 has a larger area than the chest region 402 shown in Fig. 7. The body position identification unit 313 may identify the body position of the subject 103 based on the chest region 402 in the first captured image 400.

### Actions and Effects of Second Embodiment

The configuration in which the first camera 110 and the second camera 111 are provided is effective for the abdominal examination where the accuracy of identifying the body position of the subject 103 becomes an issue in a case where there is only one camera including the first camera 110.

In a case of the abdominal examination, the movement of the upper body portion caused by breathing may occur at a plurality of close positions in the first captured image 400 regardless of whether the body position of the subject 103 is head-first or foot-first. In a case where there is one camera, there is a problem in that the body position of the subject 103 is erroneously identified.

In a case where two cameras are provided, it is possible to technically control which of the captured images of the subject 103 exhibits a relatively larger movement caused by breathing, and, for example, it is expected to improve the identification accuracy of the body position of the subject 103 in the abdominal examination.

### Third Embodiment

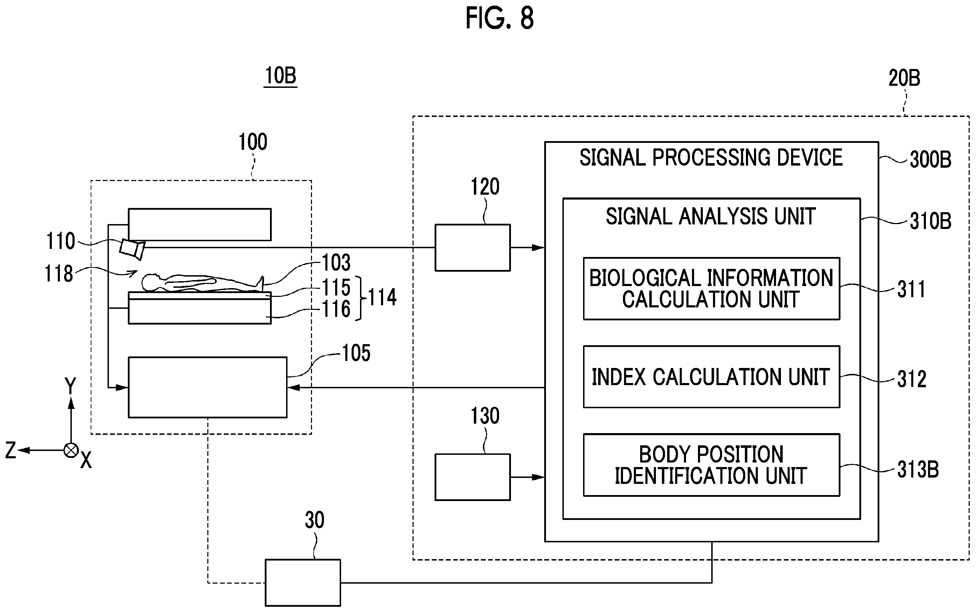

Fig. 8 is a schematic configuration diagram of a medical image capturing system according to a third embodiment. A medical image capturing system 10B shown in Fig. 8 comprises an MRI apparatus 100 and a biological information measurement system 20B. The biological information measurement system 20B comprises an examination site information acquisition unit 130.

The examination site information acquisition unit 130 acquires examination site information that is information on an examination site in the subject 103 and that includes a name of the examination site and the like. The examination site information acquisition unit 130 may acquire the examination site information registered in the MRI apparatus 100, or may acquire the examination site information manually input by the operator.

A body position identification unit 313B provided in a signal analysis unit 310B of a signal processing device 300B shown in Fig. 8 acquires the examination site information from the examination site information acquisition unit 130. The body position identification unit 313B identifies whether the body position of the subject 103 is head-first or foot-first with reference to the examination site of the subject 103 identified from the examination site information.

### Procedure of Body Position Identification Method according to Third Embodiment

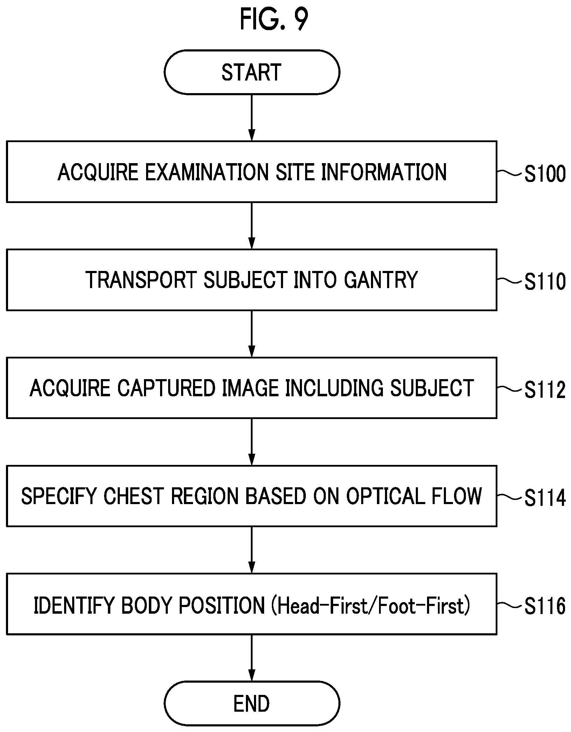

Fig. 9 is a flowchart of a body position identification method according to the third embodiment. In the body position identification method according to the third embodiment, first, step S100 is executed to acquire the examination site information of the subject 103.

In a case where the examination site information is acquired in step S100, step S110, step S112, step S114, and step S116 are executed. Step S110, step S112, and step S114 shown in Fig. 8 are respectively subjected to the same processes as step S10, step S12, and step S14 shown in Fig. 4.

In step S116, the body position of the subject 103 is identified based on the examination site information acquired in step S100 and the chest region 402 specified in step S114.

### Specific Example of Captured Image

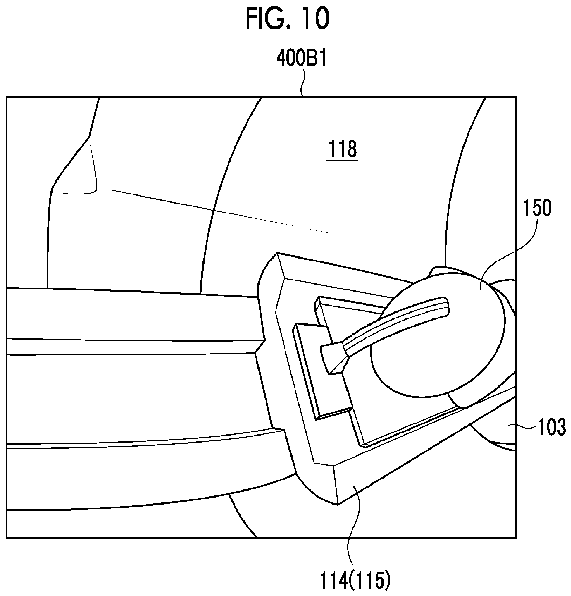

Fig. 10 is an explanatory diagram of a captured image obtained by capturing the subject undergoing a head examination using the first camera. In Fig. 10, a first captured image 400B1 in a case where the examination site is the head is shown. The first captured image 400B1 shows the subject 103 on which the receive coil for head 150 is mounted.

In a case where the examination site is the head, in a case where the body position of the subject 103 is head-first, a movement caused by the respiratory movement of the subject 103 may occur on the front side of the imaging space 118 in the first captured image 400B1. On the other hand, in a case where the body position of the subject 103 is foot-first, a movement caused by the respiratory movement of the subject 103 may occur on the rear side of the imaging space 118 in the first captured image 400B1.

A right side from the horizontal center of the first captured image 400B1 is a front side of the imaging space 118, and a left side from the horizontal center is a rear side of the imaging space 118. In a case where the body position of the subject 103 is head-first, the movement caused by the respiratory movement of the subject 103 may occur on the right side from the horizontal center of the first captured image 400B1. On the other hand, in a case where the body position of the subject 103 is foot-first, the movement caused by the respiratory movement of the subject 103 may occur on the left side from the horizontal center of the first captured image 400B1.

In a case where the examination site is the head, there may be a case where the chest region 402 is not specified, as in the first captured image 400B1. In such a case, the body position of the subject 103 may be identified as head-first. On the other hand, in a case where the chest region 402 is specified in the first captured image 400B1, the body position of the subject 103 may be identified as foot-first.

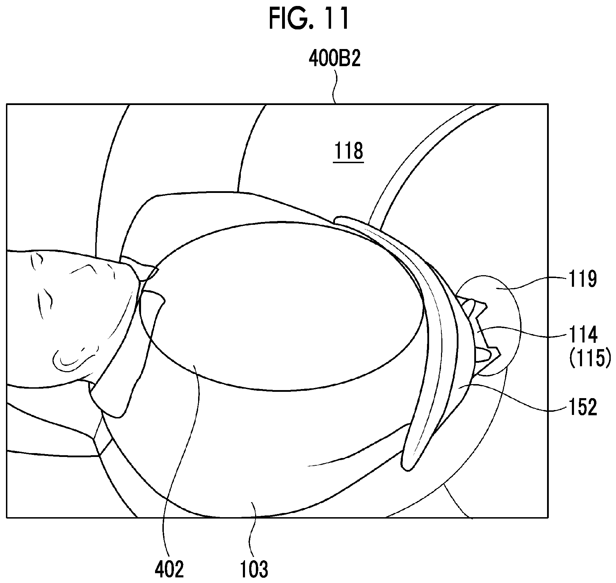

Fig. 11 is an explanatory diagram of a captured image obtained by capturing the subject undergoing a pelvic region examination using the first camera. In Fig. 11, a first captured image 400B2 in a case where the examination site is the pelvis or the periphery of the pelvis is shown. The first captured image 400B2 shows the subject 103 on which a receive coil for pelvis 152 is mounted. In addition, in the first captured image 400B2, the chest region 402 of the subject 103 is specified.

In a case where the examination site is the pelvis or the periphery of the pelvis, in a case where the body position of the subject 103 is head-first, a movement caused by the respiratory movement of the subject 103 may occur on the rear side of the imaging space 118 in the first captured image 400B2. On the other hand, in a case where the body position of the subject 103 is foot-first, a movement caused by the respiratory movement of the subject 103 may occur on the front side of the imaging space 118 in the first captured image 400B2.

A right side from the horizontal center of the first captured image 400B2 shown in Fig. 11 is a front side of the imaging space 118, and a left side from the horizontal center is a rear side of the imaging space 118.
In a case where the body position of the subject 103 is head-first, the movement caused by the respiratory movement of the subject 103 may occur on the left side from the horizontal center of the first captured image 400B1. On the other hand, in a case where the body position of the subject 103 is foot-first, the movement caused by the respiratory movement of the subject 103 may occur on the right side from the horizontal center of the first captured image 400B1.

In the first captured image 400B2 shown in Fig. 11, the chest region 402 of the subject 103 that relatively widely spreads in the vicinity of the horizontal center is specified. In such a case, the body position of the subject 103 may be identified as head-first. On the other hand, in a case where the chest region 402 of the subject 103 is specified as being relatively small on the right side from the horizontal center in the first captured image 400B2, it may be identified that the body position of the subject 103 is foot-first.

In this way, the examination site information is used to specify the region in which the chest region 402 of the subject 103 may appear in the first captured image 400B1 and the like is specified according to the body position of the subject 103.

In a case where the examination site is the abdomen, a voice prompting, such as requesting the subject 103 to move the head, is provided, and the body position of the subject 103 may be determined on the basis of the body part moved by the subject 103.

### Modification Example of Third Embodiment

The third embodiment may be combined with the second embodiment. That is, the MRI apparatus 100 shown in Fig. 8 may be provided with the second camera 111 shown in Fig. 6.

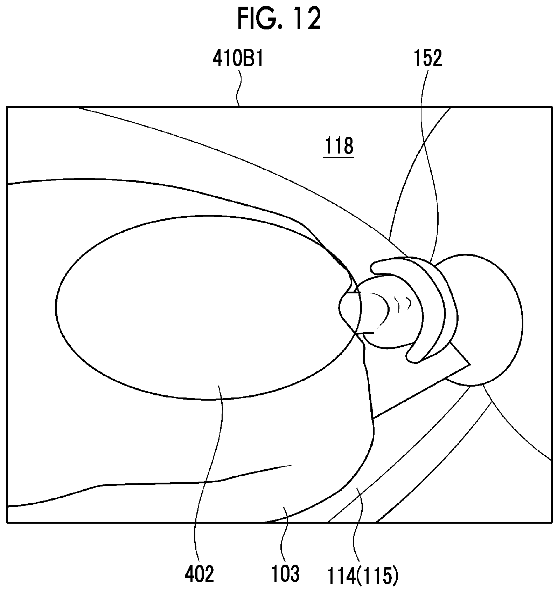

Fig. 12 is an explanatory diagram of a captured image obtained by capturing the subject undergoing the head examination using the second camera. In Fig. 12, a second captured image 410B1 in a case where the examination site is the head is shown. The second captured image 410B1 shows the subject 103 on which the receive coil for head 150 is mounted.

A left side from the horizontal center of the second captured image 410B1 shown in Fig. 12 is a front side of the imaging space 118, and a right side from the horizontal center is a rear side of the imaging space 118. In a case where the examination site is the head, in a case where the body position of the subject 103 is head-first, the movement caused by the respiratory movement of the subject 103 may occur on the left side from the horizontal center of the second captured image 410B1. On the other hand, in a case where the body position of the subject 103 is foot-first, the movement caused by the respiratory movement of the subject 103 may occur on the right side from the horizontal center of the second captured image 410B1.

Comparing the first captured image 400B1 shown in Fig. 10 with the second captured image 410B1 shown in Fig. 12, there is a difference in that the chest region 402 of the subject 103 is not specified in the first captured image 400B1, whereas the chest region 402 of the subject 103 that spreads to the left side from the horizontal center is specified in the second captured image 410B1.

From the comparison result between the first captured image 400B1 and the second captured image 410B1, it is identified that the body position of the subject 103 is head-first based on the position of the chest region 402 of the subject 103 in the second captured image 410B1.

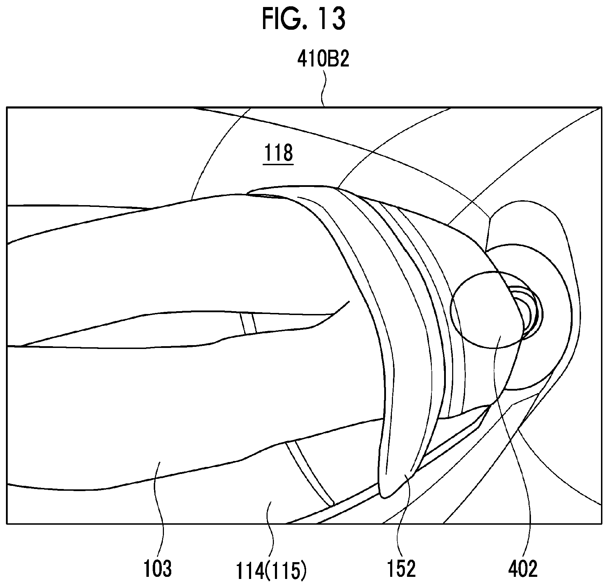

Fig. 13 is an explanatory diagram of a captured image obtained by capturing the subject undergoing the pelvic region examination using the second camera. In Fig. 13, a second captured image 410B2 in a case where the examination site is the pelvis or the periphery of the pelvis is shown. The second captured image 410B2 shows the subject 103 on which the receive coil for pelvis 152 is mounted.

A left side from the horizontal center of the second captured image 410B2 shown in Fig. 13 is a front side of the imaging space 118, and a right side from the horizontal center is a rear side of the imaging space 118. In a case where the examination site is the pelvis or the periphery of the pelvis, in a case where the body position of the subject 103 is head-first, the movement caused by the respiratory movement of the subject 103 occurs on the right side from the horizontal center of the second captured image 410B2. On the other hand, in a case where the body position of the subject 103 is foot-first, the movement caused by the respiratory movement of the subject 103 occurs on the left side from the horizontal center of the second captured image 410B2.

Comparing the first captured image 400B2 shown in Fig. 11 with the second captured image 410B2 shown in Fig. 13, it can be seen that, in the first captured image 400B2, the chest region 402 of the subject 103 having a larger area than the chest region 402 of the subject 103 specified in the second captured image 410B2 is specified.

From the comparison result between the first captured image 400B2 and the second captured image 410B2, it can be identified that the body position of the subject 103 is head-first based on the position of the chest region 402 of the subject 103 in the first captured image 400B2.

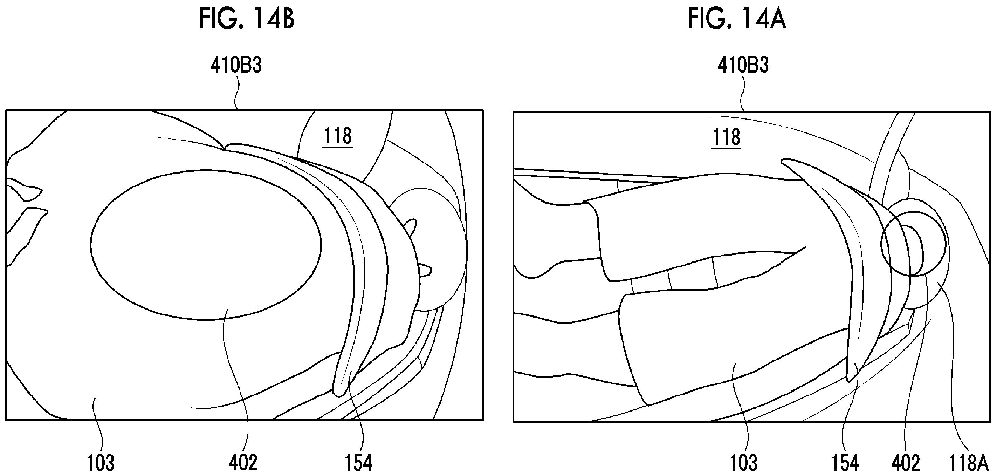

Figs. 14A and 14B are explanatory diagrams of a captured image obtained by capturing the subject undergoing an abdominal examination using the first camera and the second camera. In Fig. 14A on a right side, a first captured image 400B3 acquired by using the first camera 110 is shown. In Fig. 14B on a left side, a second captured image 410B3 acquired by using the second camera 111 is shown.

The first captured image 400B3 shown in Fig. 14A on the right side shows the subject 103 on which a receive coil for abdomen 154 is mounted. In the first captured image 400B3, the chest region 402 is specified on the right side of the horizontal center.

The second captured image 410B3 shown in Fig. 14B on the left side shows the subject 103 on which the receive coil for abdomen 154 is mounted. In the second captured image 410B3, the chest region 402 having a larger area than the chest region 402 in the first captured image 400B3 is specified on the left side of the horizontal center.

A right side from the horizontal center of the first captured image 400B3 is a front side of the imaging space 118, and a left side from the horizontal center is a rear side of the imaging space 118. A left side from the horizontal center of the second captured image 410B3 is a front side of the imaging space 118, and a right side from the horizontal center is a rear side of the imaging space 118.

In a case where the examination site is the abdomen, in a case where the body position of the subject 103 is head-first, a movement of the subject 103 caused by the respiratory movement of the subject 103 may occur on the rear side of the imaging space 118. On the other hand, in a case where the examination site is the abdomen, in a case where the body position of the subject 103 is foot-first, a movement of the subject 103 caused by the respiratory movement of the subject 103 may occur on the front side of the imaging space 118.

From the comparison result between the first captured image 400B3 and the second captured image 410B3, it can be identified that the body position of the subject 103 is foot-first based on the chest region 402 specified in the second captured image 410B3.

### Actions and Effects of Third Embodiment

The signal processing device and the body position identification method according to the third embodiment can obtain the following actions and effects.

[1] The position of the chest region 402 in the first captured image of the subject 103 is assisted to be specified based on the examination site information. As a result, the chest region 402 in the captured image of the subject 103 can be specified with high accuracy.

[2] The second captured image is acquired using the second camera 111, and the chest region 402 of the subject 103 in at least any of the first captured image or the second captured image is specified based on the comparison result between the first captured image and the second captured image. As a result, the chest region 402 in the captured image of the subject 103 can be specified with higher accuracy.

### Fourth Embodiment

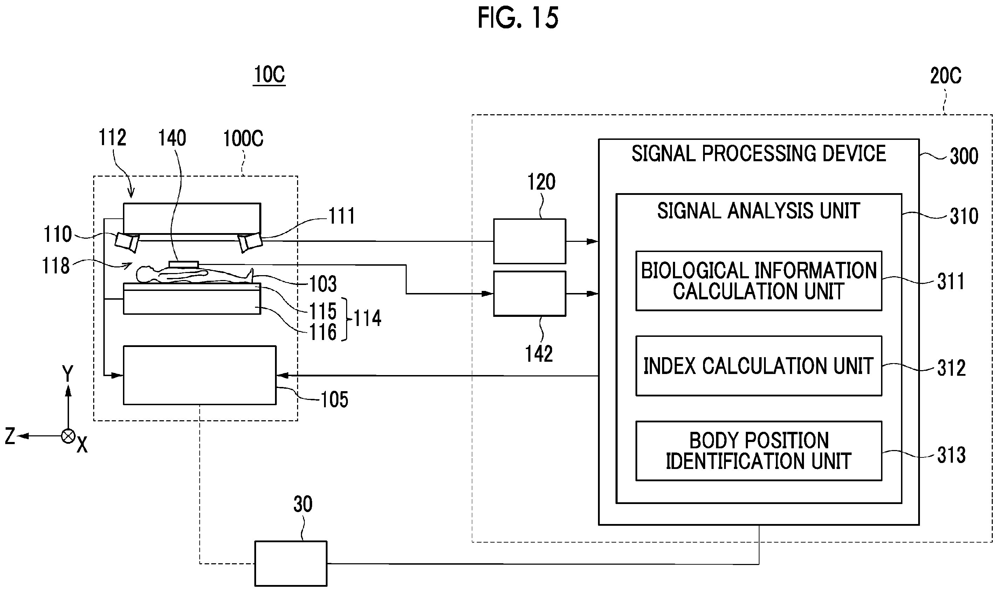

Fig. 15 is a schematic configuration diagram of a medical image capturing system according to a fourth embodiment. An MRI apparatus 100C provided in a medical image capturing system 10C shown in Fig. 15 comprises a respiratory sensor 140 that detects a respiratory movement of a subject 103 and that outputs a respiratory signal. The respiratory sensor 140 may be of a contact type that comes into contact with the subject 103, or of a noncontact type that does not come into contact with the subject 103.

In addition, a biological information measurement system 20C comprises a respiratory signal measurement device 142 that acquires the respiratory signal output from the respiratory sensor 140. The respiratory signal measurement device 142 acquires respiratory information of the subject 103 as biological information of the subject 103. The respiratory signal measurement device 142 transmits the respiratory signal to the signal processing device 300.

The biological information calculation unit 311 provided in the signal processing device 300 detects a periodically moving body movement region obtained as the optical flow of the subject 103, which is similar to the periodic respiratory signal of the subject 103, and estimates the detected body movement region as at least one of the chest or the abdomen of the subject 103.

The body position identification unit 313 identifies whether the body position of the subject 103 is head-first or foot-first based on the body movement region estimated as the chest or the like in the captured image of the subject 103.

### Procedure of Body Position Identification Method according to Fourth Embodiment

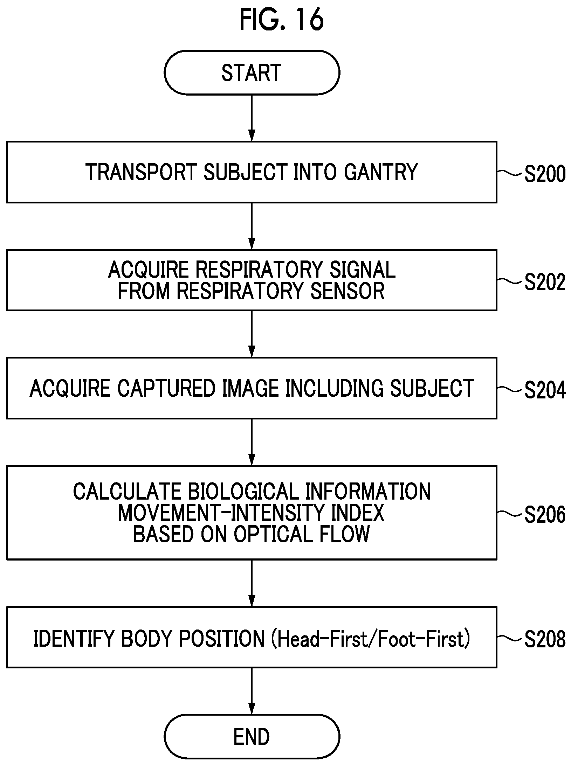

Fig. 16 is a flowchart of a body position identification method according to the fourth embodiment. In step S200, the same process as step S10 shown in Fig. 1 is executed. In step S202, the signal analysis unit 310 acquires the respiratory signal from the respiratory sensor 140 via the respiratory signal measurement device 142.

In step S204, the same process as step S12 shown in Fig. 1 is executed. In step S206, the signal analysis unit 310 calculates the optical flow based on the captured image of the subject 103.

In step S208, the signal analysis unit 310 compares the period of the optical flow calculated in step S206 with the period of the respiratory signal acquired in step S202.

In step S208, based on the comparison result between the period of the optical flow and the period of the respiratory signal, it is identified in which regions of the first captured image and the second captured image the movement with the period similar to the period of the respiratory signal occurs. Further, in step S208, the chest region 402 of the subject 103 is specified based on the identification result.

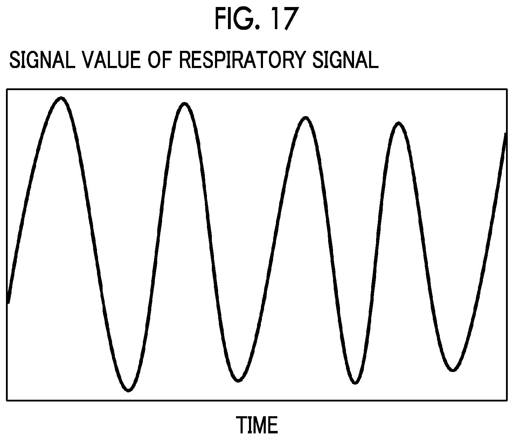

Fig. 17 is an explanatory diagram showing a waveform of the respiratory signal acquired using the respiratory sensor. In Fig. 17, a graphical format is applied to illustrate a waveform of the respiratory signal. A horizontal axis of the graph shown in Fig. 17 represents time, and a vertical axis represents a signal value of the respiratory signal.

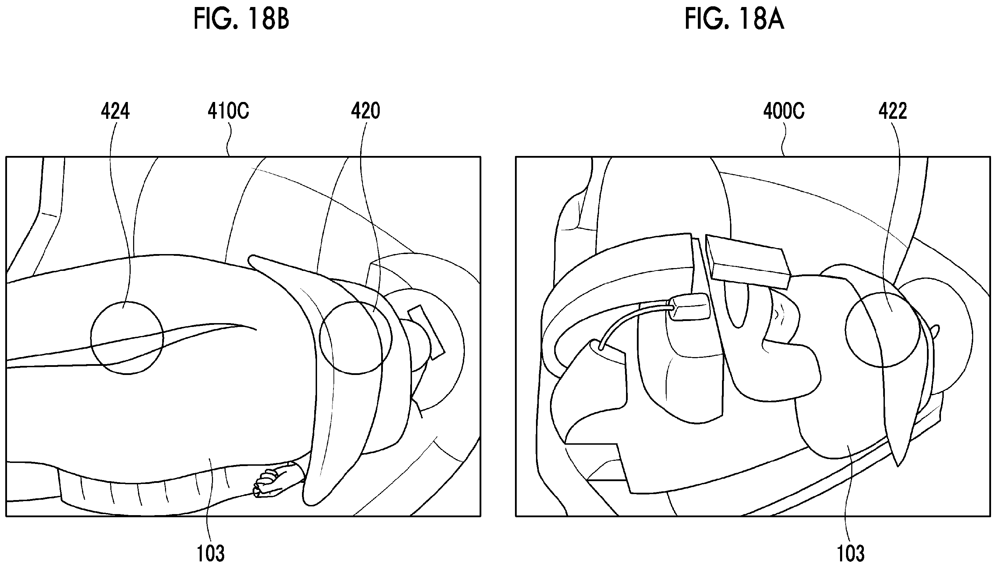

Figs. 18A and 18B are explanatory diagrams of a captured image of the subject. Fig. 18A on a right side shows a first captured image 400C that is generated by capturing the subject 103 using the first camera 110. Fig. 18B on a left side shows a second captured image 410C that is generated by capturing the subject 103 using the second camera 111.

In the second captured image 410C, a period of an optical flow of a body movement region 420 corresponding to the abdomen and a period of an optical flow of a body movement region 424 corresponding to the hip joint are calculated, and, in the first captured image 400C, a period of an optical flow of a body movement region 422 corresponding to the chest is calculated.

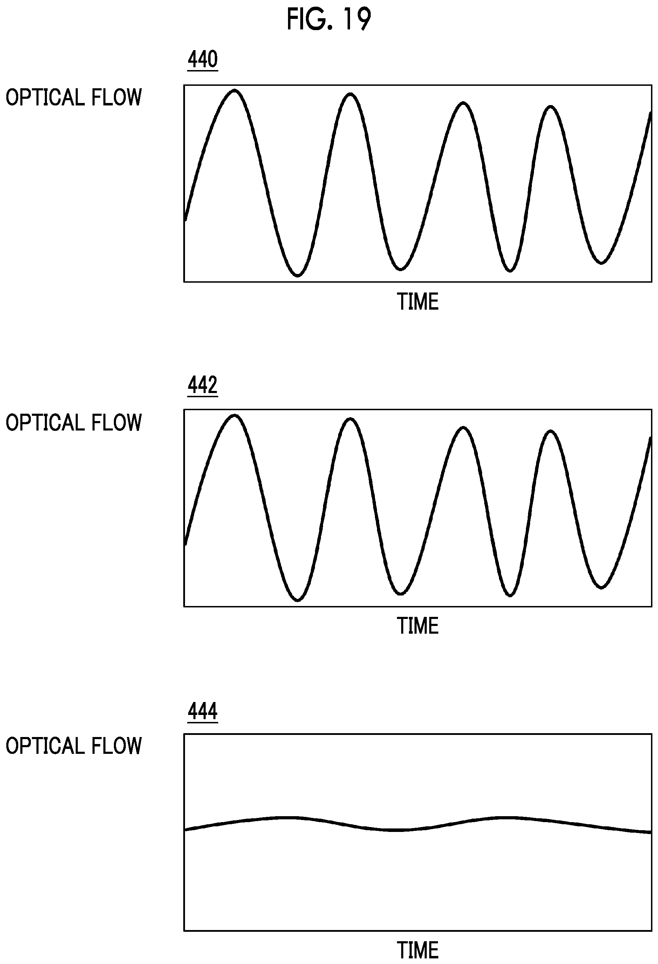

Fig. 19 is a schematic diagram showing the period of the optical flow. In Fig. 19, a graphical format is applied to schematically illustrate the period of the optical flow. A horizontal axis of the graph shown in Fig. 19 represents time, and a vertical axis represents a value of the optical flow. The value of the optical flow may be a representative value of the optical flow for each pixel. As the representative value of the optical flow, the maximum value of the optical flow for each pixel may be applied.

The optical flow value may be calculated by dividing a region to be calculated for the optical flow, and then applying an average value of the optical flow of all pixels in the region. The average value may be an arithmetic average value.

A waveform 440 represents the period of the optical flow of the body movement region 420 corresponding to the abdomen. A waveform 442 represents the period of the optical flow of the body movement region 422 corresponding to the chest. A waveform 444 represents the period of the optical flow of the body movement region 424 corresponding to the hip joint.

In step S208 shown in Fig. 16, the body position identification unit 313 shown in Fig. 15 compares the period of the respiratory signal shown in Fig. 17 with the period of the optical flow of each part shown in Fig. 19.

Further, in step S208, a captured image of the subject 103 that includes a body movement region having a period similar to the period of the respiratory signal is identified based on the comparison result, and the body position of the subject 103 is identified.

### Actions and Effects of Fourth Embodiment

In the signal processing device and the body position identification method according to the fourth embodiment, a respiratory signal representing the respiratory movement of the subject 103 is acquired by using the respiratory sensor 140. The captured image of the subject 103 in which the optical flow having a period similar to the period of the respiratory signal is calculated is specified. The body position of the subject 103 is identified based on the specified captured image. As a result, the body position of the subject 103 is identified with high accuracy.

### Overview of Procedure of MRI Imaging

Atypical imaging procedure applied to the MRI apparatus according to each of the first to fourth embodiments is generally as follows.

[Procedure 1] An operator such as a technician sets subject information, an imaging part corresponding to a target disease, an examination protocol in which an imaging type and the like are specified, and other information necessary for the examination by using the input device 332, such as a mouse and a keyboard. The operator is synonymous with a user.

The subject information includes a name of the subject, a subject code, and the like. A part or all of these pieces of information may be manually input by the operator via the input device 332 or may be read from information stored in a recording medium or the like in advance. The operator finally confirms parameters that are automatically set or input, and manually sets the parameters using the input device 332 as necessary.

[Procedure 2] The subject 103 is placed on the top plate 115 of the examination bed 114, and a receive coil such as the receive coil for head 150 is mounted on the subject 103. The operator connects a receiving-side connector of the receive coil to the nearest examination bed-side connector.

In a case where the receive coil is connected to the examination bed-side connector, the body position of the subject 103 is determined. The body position of the subject 103 includes a posture of the subject 103 and an insertion direction of the subject 103 into the gantry 112.

Examples of the posture of the subject 103 include a supine posture, a prone posture, a right lateral decubitus posture, and a left lateral decubitus posture. Examples of the posture of the subject 103 and the insertion direction of the subject 103 into the gantry 112 include head-first and foot-first.

[Procedure 3] The top plate 115 is moved to the imaging space 118 using a gantry operation panel, a foot switch, or the like, and an examination target part of the subject 103 is moved to the static magnetic field center position of the gantry 112. The gantry operation panel and the foot switch are not shown.

[Procedure 4] Scanogram imaging is executed to obtain a positioning image. In the scanogram imaging, imaging of a plurality of slices is performed for one or more cross-sections of an axial cross section, a coronal cross section, and a sagittal cross section. A slice thickness in the scanogram imaging may be set to a value larger than a slice thickness in the main imaging.

[Procedure 5] After the scanogram image capturing is completed, an image for positioning is reconstructed. The reconstructed 3D scanogram image is displayed on the display device 334. The scanogram image may include a 2D slice image in each of an axial cross section, a coronal cross section, and a sagittal cross section. The 2D slice image may include a plurality of slice images for each cross section.

[Procedure 6] The operator sets an imaging position of the main imaging based on the cross-sectional image generated from the 3D scanogram image. In the MRI apparatus 100 and the like according to the present embodiment, an imaging position including a slice position, which is a cross section position to be measured in the main imaging, is automatically calculated from the 3D scanogram image, and a recommended imaging position is presented to the operator. The operator checks the presented slice position and the like, and manually adjusts the imaging position from the input device 332 as necessary.

In addition, the operator sets imaging parameters to be applied to the main imaging. Some or all of the imaging parameters may be input by a user such as a technician or may be automatically set or input. The operator finally checks parameters that are automatically set or input, and manually sets the parameters using the input device 332 as necessary.

[Procedure 7] The main imaging is executed in accordance with the imaging parameters and the imaging position set in Procedure 6.

[Procedure 8] The main imaging is executed, and an image is reconfigured. The reconstructed image is displayed on the display device 334.

[Procedure 9] For the reconstructed image displayed on the display device 334, the operator uses the input device 332 to set a window value of a value suitable for diagnosis, and obtains an image to be used for diagnosis.

[Procedure 10] In a case where the imaging is completed, the top plate 115 is exited from the imaging space 118, and the subject 103 is exited from the gantry 112. The subject 103 is taken down from the examination bed 114, and the MRI examination is ended.

### Application Example to Program and Program Product

The body position identification method according to the embodiment may be configured as a program or a program product in which a processor or a computer including a processor realizes the functions of the respective steps.

For example, a program or a program product may be configured to cause the computer to realize a function of acquiring the captured image of the subject 103, a function of detecting the movement region of the subject 103 from the captured image of the subject 103, and a function of identifying the body position of the subject 103 based on the movement region of the subject 103.

The program or program product may be stored in a computer-readable medium that is a tangible non-transitory information storage medium, or may be provided through an information storage medium.

The present disclosure is not limited to the above-described embodiments, and various modifications can be made without departing from the scope of the technical idea of the present disclosure. In addition, each of the first to fourth embodiments can be combined as appropriate.

### Explanation of References

10: medical image capturing system
10B: medical image capturing system
14A: drawing on right side
14B: drawing on left side
18A: drawing on right side
18B: drawing on left side
20: biological information measurement system
20B: biological information measurement system
20C: biological information measurement system
30: display device
100: MRI apparatus
100A: MRI apparatus
103: subject
105: control device
110: first camera
111: second camera
112: gantry
114: examination bed
115: top plate
116: leg part
118: imaging space
119: entrance
120: biological signal measurement device
130: examination site acquisition unit
140: respiratory sensor
142: respiratory signal measurement device
150: receive coil for head
152: receive coil for pelvis
154: receive coil for abdomen
300: signal processing device
300B: signal processing device
310: signal analysis unit
310B: signal analysis unit
311: biological information calculation unit
311A: optical flow calculation unit
312: index calculation unit
312A: FFT unit
313: body position identification unit
313B: body position identification unit
322: processor
324: memory
326: storage
328: input/output interface
330: bus
332: input device
334: display device
400: first captured image
400B1: first captured image
400B2: first captured image
400B3: first captured image
402: chest region
410: second captured image
410B1: second captured image
410B2: second captured image
410B3: second captured image
420: body movement region
422: body movement region
424: body movement region
440: waveform
442: waveform
444: waveform
S10 to S16: each step of body position identification method
S100 to S116: each step of body position identification method
S200 to S208: each step of body position identification method

## Claims

1. A medical image capturing support apparatus comprising:
a processor (322); and
a memory (324) in which a program to be executed by the processor (322) is stored,
wherein the processor (322) is configured to
acquire a plurality of time-series captured images generated by capturing a subject (103),
detect, from the plurality of time-series captured images, a body movement region of the subject (103) in the captured image in which a movement of the subject (103) having periodicity and magnitude corresponding to a respiratory movement of the subject (103) occurs, and
identify a body position of the subject (103) based on the body movement region.

2. The medical image capturing support apparatus according to claim 1,
wherein the processor is configured to detect at least one of a chest or an abdomen of the subject in the captured image based on the body movement region.

3. The medical image capturing support apparatus according to claim 1 or 2,
wherein the processor (322) is configured to detect the body movement region by applying optical flow calculation processing of calculating an optical flow in the plurality of time-series captured images.

4. The medical image capturing support apparatus according to any one of claims 1 to 3,
wherein the processor (322) is configured to
acquire a plurality of time-series first captured images (400, 400B1, 400B2, 400B3) obtained by capturing the subject (103) using a first camera (110) disposed on one side of an imaging space (118),
detect a first body movement region as the body movement region from the plurality of time-series first captured images (400, 400B1, 400B2, 400B3),
acquire a plurality of time-series second captured images (410, 410B1, 410B2, 410B3) obtained by capturing the subject (103) using a second camera (111) disposed on the other side of the imaging space (118),
detect a second body movement region as the body movement region from the plurality of time-series second captured images (410, 410B, 410B2, 410B3), and
identify the body position of the subject (103) according to a comparison result between the first body movement region and the second body movement region.

5. The medical image capturing support apparatus according to any one of claims 1 to 4,
wherein the processor (322) is configured to
acquire examination site information representing an examination site,
specify a position of the body movement region in the captured image with reference to the examination site information, and
identify the body position of the subject (103) according to the position of the body movement region in the captured image.

6. The medical image capturing support apparatus according to any one of claims 1 to 5,
wherein the processor (322) is configured to
acquire a respiratory signal representing a respiratory movement of the subject (103) from a respiratory sensor (140), and
detect the body movement region based on a comparison result between periodicity of the respiratory signal and periodicity of a region in the captured image where a movement of the subject (103) having periodicity occurs.

7. The medical image capturing support apparatus according to any one of claims 1 to 6,
wherein the processor (322) is configured to identify whether the subject (103) moving to an imaging position is head-first or foot-first, as the body position of the subject (103).

8. An operation method of a medical image capturing support apparatus, the operation method comprising causing a computer functioning as the medical image capturing support apparatus to execute:
acquiring a plurality of time-series captured images generated by capturing a subject (103);
detecting, from the plurality of time-series captured images, a body movement region of the subject (103) in the captured image in which a movement of the subject (103) having periodicity and magnitude corresponding to a respiratory movement of the subject (103) occurs; and
identifying a body position of the subject (103) based on the body movement region.

9. A non-transitory, computer-readable recording medium which records thereon a program for causing a computer functioning as a medical image capturing support apparatus to execute the operation method according to claim 8.

10. A medical image capturing apparatus comprising:
an imaging unit configured to image a subject and generate imaging data of the subject;
an image reconstruction unit configured to generate reconstruction based on the imaging data; and
the medical image capturing support apparatus according to any one of claims 1 to 7.
